# EUROPÄISCHE PATENTSCHRIFT

(11) **EP 1 482 801 B1**
(45) Veröffentlichungstag und Bekanntmachung des Hinweises auf die Patenterteilung: **24.06.2009**
(21) Anmeldenummer: 03704666.1
(22) Anmeldetag: 20.02.2003
(51) Int. Cl.: A01N 47/38

(54) **HERBIZID-KOMBINATIONEN MIT SPEZIELLEN SULFONYLHARNSTOFFEN**
HERBICIDE COMBINATIONS WITH SPECIAL SULFONYLUREAS
COMBINAISONS HERBICIDES AVEC DES SULFONYLUREES PARTICULIERES

(30) Priorität: 05.03.2002 DE 10209468
(43) Veröffentlichungstag der Anmeldung: 08.12.2004
(73) Patentinhaber: Bayer CropScience AG, 40789 Monheim (DE)
(72) Erfinder: HACKER, Erwin, 65239 Hochheim (DE); BIERINGER, Hermann, 65817 Eppstein (DE); HUFF, Hans, Philipp, 65817 Eppstein (DE)
(86) Internationale Anmeldenummer: PCT/EP2003/001727
(87) Internationale Veröffentlichungsnummer: WO 2003/073855

(56) Entgegenhaltungen:
- DE-A- 19 834 629
- DE-A- 19 842 894
- ADAMCZEWSKI KAZIMIERZ ET AL: "Evaluation of iodosulfuron and amidosulfuron mixture to control broad-leaved weeds in winter and spring cereals." JOURNAL OF PLANT PROTECTION RESEARCH, Bd. 41, Nr. 2, 2001, Seiten 101-108, XP002240712 2001 ISSN: 1427-4345

## Beschreibung

Die Erfindung liegt auf dem technischen Gebiet der Pflanzenschutzmittel, die gegen Schadpflanzen z.B. in Pflanzenkulturen eingesetzt werden können und als Wirkstoffe eine Kombination von mindestens drei Herbiziden enthalten.

Aus den Druckschriften EP-A-131258 und WO 92/13845 sind Sulfonylharnstoffe und deren Salze sowie deren Verwendung als Herbizide und/oder Pflanzenwachstumsregulatoren bekannt.

Die Wirksamkeit dieser Herbizide gegen Schadpflanzen in den Pflanzenkulturen liegt auf einem hohen Niveau, hängt jedoch im allgemeinen von der Aufwandmenge, der jeweiligen Zubereitungsform, den jeweils zu bekämpfenden Schadpflanzen oder dem Schadpflanzenspektrum, den Klima- und Bodenverhältnissen, etc. ab. Ein weiteres Kriterium ist die Dauer der Wirkung bzw. die Abbaugeschwindigkeit des Herbizids. Zu berücksichtigen sind gegebenenfalls auch Veränderungen in der Empfindlichkeit von Schadpflanzen, die bei längerer Anwendung der Herbzide oder geographisch begrenzt auftreten können. Wirkungsverluste bei einzelnen Schadpflanzen lassen sich nur bedingt durch höhere Aufwandmengen der Herbizide ausgleichen, z. B. weil damit häufig die Selektivität der Herbizide verschlechtert wird oder eine Wirkungsverbesserung auch bei höherer Aufwandmenge nicht eintritt. Teilweise kann die Selektivität in Kulturen durch Zusatz von Safenem verbessert werden. Generell besteht jedoch immer Bedarf für Methoden, die Herbizidwirkung mit geringerer Aufwandmenge an Wirkstoffen zu erreichen. Eine geringere Aufwandmenge reduziert nicht nur die für die Applikation erforderliche Menge eines Wirkstoffs, sondern reduziert in der Regel auch die Menge an nötigen Formulierungshilfsmitteln. Beides verringert den wirtschaftlichen Aufwand und verbessert die ökologische Verträglichkeit der Herbizidbehandlung.

Eine Möglichkeit zur Verbesserung des Anwendungsprofits eines Herbizids kann in der Kombination des Wirkstoffs mit einem oder mehreren anderen Wirkstoffen bestehen. Allerdings treten bei der kombinierten Anwendung mehrerer Wirkstoffe nicht selten Phänomene der physikalischen und biologischen Unverträglichkeit auf, z. B. mangelnde Stabilität in einer Coformulierung, Zersetzung eines Wirkstoffes bzw. Antagonismus der Wirkstoffe. Erwünscht dagegen sind Kombinationen von Wirkstoffen mit günstigem Wirkungsprofil, hoher Stabilität und möglichst synergistisch verstärkter Wirkung, welche eine Reduzierung der Aufwandmenge im Vergleich zur Einzelapplikation der zu kombinierenden Wirkstoffe erlaubt.

Überraschenderweise wurde nun gefunden, daß bestimmte Wirkstoffe aus der Gruppe der Sulfonylharnstoffe oder deren Salzen in Kombination mit bestimmten strukturell verschiedenen Herbiziden in besonders günstiger Weise zusammenwirken, z.B. wenn sie in Pflanzenkulturen eingesetzt werden, die für die selektive Anwendung der Herbizide, gegebenenfalls unter Zusatz von Safeners, geeignet sind.

Gegenstand der Erfindung sind somit Herbizid-Kombinationen mit einem synergistisch wirksamen Gehalt an Komponenten (A), (B) und (C) wobei
(A) ein oder mehrere Herbizide aus der Gruppe der Verbindungen der Formel (I) und deren Salzen bedeutet, 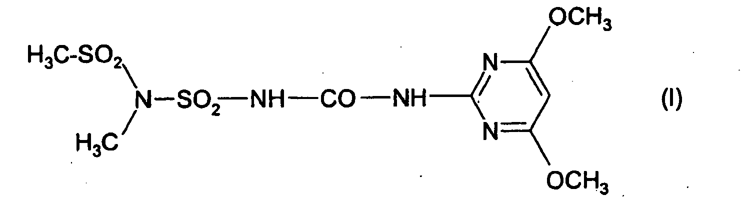
(B) ein oder mehrere Herbizide aus der Gruppe der Verbindungen der Formel (II) und deren Salzen bedeutet, 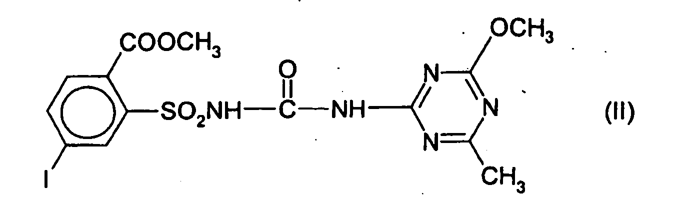
(C) bedeutet ein oder mehrere selektiv in einigen monokotylen Kulturen gegen monokotyle und/oder dikotyle Schadpflanzen wirksame Herbizide aus der Gruppe der Herbizide, bestehend aus (Angabe mit dem "common name" und einer Referenzstelle, z.B. aus "The Pesticide Manual" 12th Ed., British Crop Protection Council 2000, abgekürzt "PM")
   (C1) Flucarbazone, insbesondere auch umfassend dessen Salze wie das Natriumsalz (PM, S. 427-428). z.B. 4,5-dihydro-3-methoxy-4-methyl-5-oxo-N-(2-trifluoromethoxyphenylsulfonyl)-1H-1,2,4-triazole-1-carboxamid Natrium Salz, (Aufwandmenge im allgemeinen: 1 - 500g AS/ha, vorzugsweise 5 - 200g AS/ha; Aufwandmengenvefiältnis (A + B) : C im allgemeinen = 1: 100 -100 : 1, vorzugsweise 1 : 20 - 50 : 1);
   (C2) Procarbazone (BAY MKH 6561), insbesondere auch umfassend dessen Ester und Salze wie das Natriumsalz (Z. PflKrankh. PflSchutz, Sonderh. XVII, 545-553 (2000)), z.B. Methyl 2-({[(4-methyl-5-oxo-3-propoxy-4,5-dihydro-1 H-1,2,4-triazol-1-yl)carbonyl]amino}sulfonyl)benzoat Natrium Salz, (Aufwandmenge im allgemeinen: 1 - 500g AS/ha, vorzugsweise 5 - 200g AS/ha; Aufwandmengenverhältnis (A + B): C im allgemeinen = 1 : 100 - 100 : 1, vorzugsweise 1 : 20 -10 : 1);
   (C5) Tritosulfuron, insbesondere auch umfassend dessen Salze wie das Natriumsalz (AG Chem, New Compound Review (publ. Agranova), Vol. 17,1999, S. 24), z.B. N-[[[4'-Methoxy-6-(trifluoromethyl)-1,3,5-triazin-2-yl]amino]carbonyl]-2-trifluoromethylbenzolsulfonamid), (Aufwandmenge: 1 - 500 g AS/ha, vorzugsweise 5 - 200g AS/ha; Aufwandmengenverhältnis (A + B): C im allgemeinen = 1 : 100 - 100 : 1, vorzugsweise 1 : 20 - 25 : 1);
   (C13) Flufenacet, insbesondere auch umfassend dessen Salze wie das Natriumsalz (PM, S. 434-435), z.B. 4'-Fluoro-N-isopropyl-2-(5-trifluoromethyl-1,3,4-thiadiazol-2-yloxy)acetanilid, (Aufwandmenge im allgemeinen: 50 - 5000g AS/ha, vorzugsweise 150 - 2000g AS/ha; Aufwandmengenverhältnis (A + B) : C im allgemeinen = 1 : 1000 - 2 : 1, vorzugsweise 1 : 200 - 1 : 3);
   (C19) Sulfosulfuron, insbesondere auch umfassend dessen Salze wie das Natriumsalz (PM, S. 853-854), z.B. 1-(4,6-Dimethoxypyrimidin-2-yl)-3-(2-ethylsulfonylimidazo[1,2-a]pyridin-3-yl)sulfonylhamstoff, (Aufwandmenge im allgemeinen: 1 - 500g AS/ha, vorzugsweise 5-200g AS/ha; Aufwandmengenverhältnis (A + B) : C im allgemeinen = 1 : 100 - 100:1, vorzugsweise 1 : 20-10:1); und
   (C20) Flupyrsulfuron-methyl und dessen Salze wie das Natriumsalz, z.B. Flupyrsulfuron-methyl-Natrium (PM, S. 447-448), z.B. Methyl 2-(4,6-dimethoxypyrimidin-2-ylcarbamoylsulfamoyl)-6-trifluoromethylnicotinat Natrium Salz, (Aufwandmenge: 1 - 500 g AS/ha, vorzugsweise 5 - 200g AS/ha; Aufwandmengenverhältnis (A + B) : C im allgemeinen = 1 : 100 -100 : 1, vorzugsweise 1 : 20 -10 : 1).
   (C30) MCPB, (PM, S. 586-588), z.B. 4-(4-Chlor-2-methyl-phenoxy)-butansäure oder deren Salze (z.B. Natriumsalz), (Aufwandmenge: 100 - 5000 g AS/ha, vorzugsweise 200 - 3000 AS/ha; Aufwandmengenverhältnis (A + B) : C im allgemeinen = 1 : 500 -1:1, vorzugsweise 1 : 300 - 1 : 4);

Wenn im Rahmen dieser Beschreibung die Kurzform des "common name" eines Wirkstoffs verwendet wird, so sind damit jeweils alle gängigen Derivate, wie die Ester und Salze, und Isomere, insbesondere optische Isomere umfasst, insbesondere die handelsübliche Form bzw. Formen. Wird mit dem "common name" ein Ester oder Salz bezeichnet, so sind damit auch jeweils alle anderen gängigen Derivate wie andere Ester und Salze, die freien Säuren und Neutralverbindungen, und Isomere, insbesondere optische Isomere umfasst, insbesondere die handelsübliche Form bzw. Formen. Die angegebenen chemischen Verbindungsnamen bezeichnen zumindest eine der von dem "common name" umfaßten Verbindungen, häufig eine bevorzugte Verbindung. Bei Sulfonamiden wie Sulfonylharnstoffen sind mit Salzen auch die umfaßt, die durch Austausch eines Wasserstoffatoms an der Sulfonamidgruppe durch ein Kation entstehen.

Die Herbizide C1 - C2, C13, C19 und C20 eignen sich zur Bekämpfung monkotyler und dikotyler Schadpflanzen. Die Herbizide C30 eignen sich insbesondere zur Bekämpfung dikotyler Schadpflanzen.

Die erfindungsgemäßen Herbizid-Kombinationen weisen einen herbizid wirksamen Gehalt an Komponenten (A), (B) und (C) auf und können weitere Komponenten enthalten, z. B. agrochemische Wirkstoffe anderer Art und/oder im Pflanzenschutz übliche Zusatzstoffe und/oder Formulierungshilfsmittel, oder zusammen mit diesen eingesetzt werden. Es sind Herbizid-Kombinationen bevorzugt, die einen synergistisch wirksamen Gehalt an Komponenten (A), (B) und (C) aufweisen.

Die erfindungsgemäßen Herbizid-Kombinationen weisen synergistische Wirkungen auf. Die synergistischen Wirkungen können z.B. bei gemeinsamer Ausbringung der Wirkstoffe (A), (B) und (C) beobachtet werden, sie können jedoch häufig auch bei zeitlich versetzter Anwendung (Splitting) festgestellt werden. Möglich ist auch die Anwendung der einzelnen Herbizide oder der Herbizid-Kombinationen in mehreren Portionen (Sequenzanwendung), z. B. Anwendungen im Vorauflauf, gefolgt von Nachauflauf-Applikationen oder frühe Nachauflaufanwendungen, gefolgt von Applikationen im mittleren oder späten Nachauflauf. Bevorzugt ist dabei die gemeinsame oder die zeitnahe Anwendung der Wirkstoffe der erfindungsgemäßen Herbizid-Kombination.

Die synergistischen Effekte erlauben eine Reduktion der Aufwandmengen der Einzelwirkstoffe, eine höhere Wirkungsstärke bei gleicher Aufwandmenge, die Kontrolle bislang nicht erfasster Arten (Lücken), eine Ausdehnung des Anwendungszeitraums und/oder eine Reduzierung der Anzahl notwendiger Einzelanwendungen und - als Resultat für den Anwender - ökonomisch und ökologisch vorteilhaftere Unkrautbekämpfungssysteme.

Die genannte Formel (I) umfaßt alle Stereoisomeren und deren Gemische, insbesondere auch racemische Gemische, und - soweit Enantiomere möglich sind - die jeweils biologisch wirksamen Enantiomere. Verbindungen der Formel (I) und ihre Salze sowie ihre Herstellung sind z.B. beschrieben in EP-A-131258 und US 4,718,937. Bevorzugte Verbindungen der Formel (I) und deren Salze sind 1-(4,6-dimethoxypyrimidin-2-yl)-3-mesyl-(methyl)sulfamoyl-hamstoff (Amidosulfuron, A1) und dessen Salze wie das Natriumsalz (Amidosulfuron-Natrium, A2) (siehe z.B. EP-A-131258 und PM, S. 29-30).

Die genannte Formel (II) umfaßt alle Stereoisomeren und deren Gemische, insbesondere auch racemische Gemische, und - soweit Enantiomere möglich sind - die jeweils biologisch wirksamen Enantiomere. Verbindungen der Formel (II) und ihre Salze sowie ihre Herstellung sind z.B. beschrieben in WO 92/13845. Bevorzugte Verbindungen der Formel (II) und deren Salze sind 3-(4-Methoxy-6-methyl-1,3,5-triazin-2-yl)-1-(2-methoxycarbonyl-5-iod-phenyl-sulfonyl)-harnstoff (lodosulfuron-methyl, B1) und dessen Salze wie das Natriumsalz (lodosulfuron-methyl-Natrium, B2) (siehe z.B. WO 92/13845 und PM, S. 547-548).

Die genannten Wirkstoffe der Formel (I) und (II) und deren Salze können das Enzym Acetolactatsynthase (ALS) und damit die Proteinsynthese in Pflanzen hemmen. Die Aufwandmenge der Wirkstoffe der Formel (I) und (II) und deren Salze kann in weiten Bereichen variieren, beispielsweise zwischen 0,001 und 0,5 kg AS/ha. Soweit in dieser Beschreibung die Abkürzung AS/ha verwendet wird, bedeutet dies "Aktivsubstanz pro Hektar", bezogen auf 100%igen Wirkstoff. Bei Anwendungen mit Aufwandmengen von 0,01 bis 0,3 kg AS/ha der Wirkstoffe der Formel (I) und (II) und deren Salzen, vorzugsweise der Wirkstoffe (A1), (A2), (B1) und (B2) wird im Vor- und Nachauflaufverfahren ein relativ breites Spektrum an annuellen und perennierenden Unkräutern, Ungräsem sowie Cyperaceen bekämpft. Bei den erfindungsgemäßen Kombinationen liegen die Aufwandmengen in der Regel niedriger, z. B. im Bereich von 0,1 bis 200 g AS/ha, vorzugsweise 0,5 bis 120 g AS/ha.

Die Wirkstoffe können in der Regel als wasserlösliches Spritzpulver (WP), wasserdispergierbares Granulat (WDG), wasseremulgierbares Granulat (WEG), Suspoemulsion (SE) oder Ölsuspensionskonzentrat (SC) formuliert werden.

Die im allgemeinen verwendeten Aufwandmengenverhältnisse (A + B) /C sind vorstehend angegeben und bezeichnen das Gewichtsverhältnis der Komponenten (A + B) und C zueinander. Das Gewichtsverhältnis der Komponenten A und B zueinanender beträgt dabei im allgemeinen 100 : 1 - 1 : 10, vorzugsweise 20 : 1 - 1:1.

Zur Anwendung der Wirkstoffe der Formel (I) und (II) und deren Salzen in Pflanzenkulturen kann es je nach Pflanzenkultur zweckmäßig sein, ab bestimmter Aufwandmengen einen Safener zu applizieren, um eventuelle Schäden an der Kulturpflanze zu reduzieren oder zu vermeiden. Beispiele für geeignete Safener sind solche, die in Kombination mit Sulfonylhamstoff-Herbiziden, vorzugsweise, Phenylsulfonylharnstoffen Safenerwirkung entfalten. Geeignete Safener sind z.B. aus WO-A-96/14747 und der dort zitierten Literatur bekannt.

Folgende Gruppen von Verbindungen sind beispielsweise als Safener für die oben erwähnten herbiziden Wirkstoffe (A) und (B) geeignet:
a) Verbindungen vom Typ der Dichlorphenylpyrazolin-3-carbonsäure (S1), vorzugsweise Verbindungen wie 1-(2,4-Dichlorphenyl)-5-(ethoxycarbonyl)-5-methyl-2-pyrazolin-3-carbonsäureethylester (S1-1, Mefenpyr-diethyl, PM, S. 594 - 595), und verwandte Verbindungen, wie sie z.B. in der WO 91/07874 und PM (S.594-595) beschrieben sind,
b) Derivate der Dichlorphenylpyrazolcarbonsäure, vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4), 1-(2,4-Dichlorphenyl)-5-phenyl-pyrazol-3-carbonsäureethylester (S1-5) und verwandte Verbindungen, wie sie in EP-A-333 131 und EP-A-269 806 beschrieben sind.
c) Verbindungen vom Typ der Triazolcarbonsäuren (S1), vorzugsweise Verbindungen wie Fenchlorazol, z.B. 1-(2,4-Dichlorphenyl)-5-trichlormethyl-(1H)-1,2,4-triazol-3-carbonsäureethylester (S1-6), und verwandte Verbindungen (siehe EP-A-174 562 und EP-A-346 620);
d) Verbindungen vom Typ der 5-Benzyl- oder 5-Phenyl-2-isoxazolin-3-carbonsäure, oder der 5,5-Diphenyl.2-isoxazolin-3-carbonsäure vorzugsweise Verbindungen wie 5-(2,4-Dichlorbenzyl)-2-isoxazolin-3-carbonsäureethylester (S1-7) oder 5-Phenyl-2-isoxazolin-3-carbonsäureethylester (S1-8) und verwandte Verbindungen, wie sie z.B. in WO 91/08202 beschrieben sind, bzw. der 5,5-Diphenyl-2-isoxazolin-3-carbonsäureethylester (S1-9, Isoxadifen-ethyl) oder -n-propylester (S1-10) oder der 5-(4-Fluorphenyl)-5-phenyl-2-isoxazolin-3-carbonsäureethylester (S1-11), wie sie in der Patentanmeldung (WO-A-95/07897) beschrieben sind.
e) Verbindungen vom Typ der 8-Chinolinoxyessigsäure (S2), vorzugsweise (5-Chlor-8-chinolinoxy)-essigsäure-(1-methyl-hex-1-yl)-ester (S2-1, Cloquintocet-mexyl, z.B. PM (S. 195-196), (5-Chlor-8-chinolinoxy)-essigsäure-(1,3-dimethyl-but-1-yl)-ester (S2-2), (5-Chlor-8-chinolinoxy)-essigsäure-4-allyl-oxy-butylester (S2-3), (5-Chlor-8-chinolinoxy)-essigsäure-1-allyloxy-prop-2-ylester (S2-4), (5-Chlor-8-chinolinoxy)-essigsäureethylester (S2-5), (5-Chlor-8-chinolinoxy)-essigsäuremethylester (S2-6), (5-Chlor-8-chinolinoxy)-essigsäureallylester(S2-7), (5-Chlor-8-chinolinoxy)-essigsäure-2-(2-propyliden-iminoxy)-1-ethylester (S2-8), (5-Chlor -8-chinolinoxy)-essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind.
f) Verbindungen vom Typ der (5-Chlor-8-chinolinoxy)-malonsäure, vorzugsweise Verbindungen wie (5-Chlor-8-chinolinoxy)-malonsäurediethylester, (5-Chlor-8-chinolinoxy)-malonsäurediallylester, (5-Chlor-8-chinolinoxy)-malonsäure-methyl-ethylester und verwandte Verbindungen, wie sie in EP-A-0 582 198 beschrieben sind.
g) Wirkstoffe vom Typ der Phenoxyessig- bzw. -propionsäurederivate bzw. der aromatischen Carbonsäuren, wie z.B. 2,4-Dichlorphenoxyessigsäure(ester) (2,4-D), 4-Chlor-2-methyl-phenoxy-propionester (Mecoprop), MCPA oder 3,6-Dichlor-2-methoxy-benzoesäure(ester) (Dicamba).

Für Wirkstoffe der Gruppe (C) sind die obengenannten Safener vielfach ebenfalls geeignet. Darüber hinaus eignen sich folgende Safener für die erfindungsgemäßen Herbizid-Kombinationen:
h) Wirkstoffe vom Typ der Pyrimidine, wie "Fenclorim" (PM, S. 386-387) (= 4,6-Dichlor-2-phenylpyrimidin),
i) Wirkstoffe vom Typ der Dichloracetamide, die häufig als Vorauflaufsafener (bodenwirksame Safener) angewendet werden, wie z.B.
   "Dichlormid" (PM, S. 270-271) (= N,N-Diallyi-2,2-dichloracetamid), AR-29148" (= 3-Dichloracetyl-2,2,5-trimethyl-1,3-oxazolidon von der Firma Stauffer),
   "Benoxacor" (PM, S. 74-75) (= 4-Dichloracetyl-3,4-dihydro-3-methyl-2H-1,4-benzoxazin).
   APPG-1292" (= N-Allyl-N[(1,3-dioxolan-2-yl)-methyl]dichloracetamid von der Firma PPG Industries),
   ADK-24" (= N-Allyl-N-[(allylaminocarbonyl)-methyl]-dichloracetamid von der Firma Sagro-Chem),
   AAD-67" oder AMON 4660" (= 3-Dichloracetyl-1-oxa-3-aza-spiro[4,5]decan von der Firma Nitrokemia bzw. Monsanto),
   "Diclonon" oder ABAS145138" oder ALAB145138" (= (= 3-Dichloracetyl-2,5,5-trimethyl-1,3-diazabiclyco[4.3.0]nonan von der Firma BASF) und
   "Furilazol" oder AMON 13900" (siehe PM, S. 482-483) (= (RS)-3-Dichloracetyl-5-(2-furyl)-2,2-dimethyloxazolidon)
j) Wirkstoffe vom Typ der Dichloracetonderivate, wie z.B.
   AMG 191" (CAS-Reg. Nr. 96420-72-3) (= 2-Dichlormethyl-2-methyl-1,3-dioxolan von der Firma Nitrokemia),
k) Wirkstoffe vom Typ der Oxyimino-Verbindungen, die als Saatbeizmittel bekannt sind, wie z.B.
   "Oxabetrinil" (PM, S. 689) (= (Z)-1,3-Dioxolan-2-ylmethoxyimino(phenyl)acetonitril), das als Saatbeiz-Safener gegen Schäden von Metolachlor bekannt ist,
   "Fluxofenim" (PM, S. 467-468) (= 1-(4-Chlorphenyl)-2,2,2-triftuor-1-ethanon-O-(1,3-dioxolan-2-ylmethyl)-oxim, das als Saatbeiz-Safener gegen Schäden von Metolachlor bekannt ist, und
   "Cyometrinil" oder A-CGA-43089" (PM, S. 983) (= (Z)-Cyanomethoxyimino (phenyl)acetonitril), das als Saatbeiz-Safener gegen Schäden von Metolachlor bekannt ist,
l) . Wirkstoffe vom Typ der Thiazolcarbonsäureester, die als Saatbeizmittel bekannt sind, wie z.B.
   "Flurazol" (PM, S. 450-451) (= 2-Chlor-4-trifluormethyl-1,3-thiazol-5-carbonsäurebenzylester), das als Saatbeiz-Safener gegen Schäden von Alachlor und Metolachlor bekannt ist,
m) Wirkstoffe vom Typ der Napthalindicarbonsäurederivate, die als Saatbeizmittel bekannt sind, wie z.B.
   "Naphthalic anhydrid" (PM, S. 1009-1010) (= 1,8-Naphthalindicarbonsäureanhydrid), das als Saatbeiz-Safener für Mais gegen Schäden von Thiocarbamatherbiziden bekannt ist,
n) Wirkstoffe vom Typ Chromanessigsäurederivatre, wie z.B.
   ACL 304415" (CAS-Reg. Nr. 31541-57-8) (= 2-84-Carboxy-chroman-4-yl)-essigsäure von der Firma American Cyanamid),
o) Wirkstoffe, die neben einer herbiziden Wirkung gegen Schadpflanzen auch Safenerwirkung an Kulturpflanzen aufweisen, wie z.B.
   "Dimepiperate" oder AMY-93" (PM, S. 302-303) (= Piperidin-1-thiocarbonsäure-S-1-methyl-1-phenylethylester),
   "Daimuron" oder ASK 23" (PM, S. 247) (= 1-(1-Methyl-1-phenylethyl)-3-p-tolylhamstoff),
   "Cumyluron" = AJC-940" (= 3-(2-Chlorphenylmethyl)-1-(1-methyl-1-phenylethyl)-harnstoff, siehe JP-A-60087254),
   "Methoxyphenon" oder ANK 049" (= 3,3'-Dimethyl-4-methoxybenzophenon),
   "CSB" (= 1-Brom-4-(chlormethylsulfonyl)-benzol) (CAS-Reg. Nr. 54091-06-4 von Kumiai).

Die Herbizide (A) und (B) sind, gegebenenfalls in Gegenwart von Safenem (z.B. ist die Kombination (A1) + (B2) + (S1-1) kommerziell erhältlich als Hoestar^{®} Super), zur Bekämpfung von Schadpflanzen in Pflanzenkulturen geeignet, beispielsweise in wirtschaftlich bedeutenden Kulturen wie Getreide (z.B. Weizen, Gerste, Roggen, Hafer, Reis, Mais, Hirse), Zuckerrübe, Zuckerrohr, Raps, Baumwolle und Soja. Von besonderem Interesse ist dabei die Anwendung in monokotylen Kulturen wie Getreide, insbesondere Weizen, Gerste, Roggen, Hafer und Kreuzungen davon wie Triticale. Für die Kombinationen (A)+(B)+(C) sind diese Kulturen ebenfalls bevorzugt.

Erfindungsgemäß umfasst sind auch solche Herbizid- Kombinationen, die neben den Komponenten (A), (B) und (C) noch ein oder mehrere weitere agrochemische Wirkstoffe anderer Struktur enthalten, wie Herbizide, Insektizide, Fungizide oder Safener. Für solche Kombinationen gelten die nachstehend insbesondere für erfindungsgemäße Kombinationen (A) + (B) + (C) erläuterten bevorzugten Bedingungen in erster Linie ebenfalls, sofern darin die erfindungsgemäßen Kombinationen (A) + (B) + (C) enthalten sind und bezüglich der betreffenden Kombination (A) + (B) + (C).

Von besonderem Interesse ist die Anwendung von herbiziden Mitteln mit einem Gehalt an folgenden Verbindungen (A) + (B) + (C):
(A1) +(B1)+(C1), (A1) + (B1) + (C2), (A1) + (B1) + (C5), (A1) + (B1) + (C13), (A1) + (B1) + (C19), (A1) + (B1) + (C20), (A1) + (B1) + (C30),
(A1) + (B2) + (C1), (A1) + (B2) + (C2), (A1) + (B2) + (C5), (A1) + (B2) + (C13), (A1) + (B2) + (C19), (A1) + (B2) + (C20), (A1) + (B2) + (C30),
(A2) + (B1) + (C1), (A2) + (B1) + (C2), (A2)+ (B1) + (C5), (A2) + (B1) + (C13), (A2) + (B1) + (C19), (A2) + (B1) + (C20), (A2) + (B1) + (C30),
(A2) + (B2) + (C1), (A2) + (B2) + (C2), (A2)+ (B2) + (C5), (A2) + (B2) + (C13), (A2) + (B2) + (C19), (A2) + (B2) + (C20), (A2) + (B2) + (C30),
(A1) + (A2) + (B1) + (B2) + (C1), (A1) + (A2) + (B1) + (B2) + (C2), (A1) + (A2) + (B1) + (B2) + (C5), (A1) + (A2) + (B1) + (B2)+(C13), (A1) + (A2) + (B1) + (B2) + (C19), (A1) + (A2) + (B1) + (B2) + (C20), (A1) + (A2) + (B1) + (B2) + (C30).

Darüber hinaus kann jede der oben genannten Herbizid-Kombinationen noch einen oder mehrere Safener enthalten, insbesondere einen Safener wie Mefenpyr-diethyl (S1-1), Isoxadifen-ethyl (S1-9) und Cloquintocet-mexyl (S2-1). Dabei sind die oben genannten Aufwandmengenbereiche und Aufwandmengenverhältnisse jeweils bevorzugt. Beispiele hierfür sind die nachfolgend genannten Herbizid-Kombinationen.
(A1) + (B1) + (C1) + (S1-1), (A1) + (B1) + (C2) + (S1-1), (A1) + (B1) + (C5) + (S1-1), (A1) + (B1) + (C13) + (S1-1),
(A1) + (B1) + (C19) + (S1-1), (A1) + (B1) + (C20) + (S1-1), (A1) + (B1) + (C30) + (S1-1);
(A1) + (B2) + (C1) + (S1-1), (A1) + (B2) + (C2) + (S1-1), (A1) + (B2)+ (C5) + (S1-1), (A1) + (B2) + (C13) + (S1-1), (A1) + (B2) + (C19) + (S1-1), (A1) + (B2) + (C20) + (S1-1), (A1) + (B2) + (C30) + (S1-1);
(A2) +(B1) + (C1) + (S1-1), (A2) + (B1) + (C2) + (S1-1), (A2) + (B1) + (C5) + (S1-1), (A2) + (B1) + (C13) + (S1-1), (A2) + (B1) + (C19) + (S1-1), (A2) + (B1) + (C20) + (S1-1), (A2) + (B1) + (C30) + (S1-1);
(A2) + (B2) + (C1) + (S1-1), (A2) + (B2) + (C2) + (S1-1), (A2) + (B2) + (C5) + (S1-1), (A2) + (B2) + (C13) + (S1-1), (A2) + (B2) + (C19) + (S1-1), (A2) + (B2) + (C20) + (S1-1), (A2) + (B2) + (C30) + (S1-1);
(A1) + (A2) + (B1) + (B2) + (C1) + (S1-1), (A1) + (A2) + (B1) + (B2) + (C2) + (S1-1), (A1) + (A2) + (B1) + (B2) + (C5) + (S1-1), (A1) + (A2) + (B1) + (B2) + (C13) + (S1-1), (A1) + (A2) + (B1) + (B2) + (C19) + (S1-1), (A1) + (A2) + (B1) + (B2) + (C20) + (S1-1), (A1) + (A2) + (B1) + (B2) + (C30)+ (S1-1);
(A1) + (B1) + (C1) + (S1-9), (A1) + (B1) + (C2) + (S1-9), (A1) + (B1) + (C5) + (S1-9), (A1) + (B1) + (C13) + (S1-9), (A1) + (B1) + (C19) + (S1-9), (A1) + (B1) + (C20) + (S1-9), (A1) + (B1) + (C30) + (S1-9);
(A1) + (B2) + (C1) + (S1-9), (A1) + (B2) + (C2) + (S1-9), (A1) + (B2) + (C5) + (S1-9), (A1) + (B2) + (C13) + (S1-9), (A1) + (B2) + (C19) + (S1-9), (A1) + (B2) + (C20) + (S1-9), (A1) + (B2) + (C30) + (S1-9);
(A2) + (B1) + (C1) + (S1-9), (A2) + (B1) + (C2) + (S1-9), (A2) + (B1) + (C5) + (S1-9), (A2) + (B1) + (C13) + (S1-9), (A2) + (B1) + (C19) + (S1-9), (A2) + (B1) + (C20) + (S1-9), (A2) + (B1) + (C30) + (S1-9);
(A2) + (B2) + (C1) + (S1-9), (A2) + (B2) + (C2) + (S1-9), (A2) + (B2) + (C5) + (S1-9), (A2) + (B2) + (C13) + (S1-9), (A2) + (B2) + (C19) + (S1-9), (A2) + (B2) + (C20) + (S1-9), (A2) + (B2) + (C30) + (S1-9);
(A1) + (A2) + (B1) + (B2) + (C1) + (S1-9), (A1) + (A2) + (B1) + (B2) + (C2) + (S1-9), (A1) + (A2) + (B1) + (B2) + (C5) + (S1-9), (A1) + (A2) + (B1) + (B2) + (C13) + (S1-9), (A1) + (A2) + (B1) + (B2) + (C19) + (S1-9), (A1) + (A2) + (B1) + (B2) + (C20) + (S1-9), (A1) + (A2) + (B1) + (B2) + (C30) + (S1-9);
(A1) + (B1) + (C1) + (S2-1), (A1) + (B1) + (C2) + (S2-1), (A1) + (B1) + (C5) + (S2-1), (A1) + (B1) + (C13) + (S2-1), (A1) + (B1) + (C19) + (S2-1), (A1) + (B1) + (C20) + (S2-1), (A1) + (B1) + (C30) + (S2-1);
(A2) + (B1) + (C1) + (S2-1), (A2) + (B1) + (C2) + (S2-1), (A2) + (B1) + (C5) + (S2-1), (A2) + (B1) + (C13) + (S2-1), (A2) + (B1) + (C19) + (S2-1), (A2) + (B1) + (C20) + (S2-1), (A2) + (B1) + (C30) + (S2-1);
(A2) + (B2) + (C1) + (S2-1), (A2) + (B2) + (C2) + (S2-1), (A2) + (B2) + (C5) + (S2-1), (A2) + (B2) + (C13) + (S2-1), (A2) + (B2) + (C19) + (S2-1), (A2) + (B2) + (C20) + (S2-1), (A2) + (B2) + (C30) + (S2-1);
(A1) + (A2) + (B1) + (B2) + (C1) + (S2-1), (A1) + (A2) + (B1) + (B2) + (C2) + (S2-1), (A1) + (A2) + (B1) + (B2) + (C5) + (S2-1), (A1) + (A2) + (B1) + (B2) + (C13) + (S2-1), (A1) + (A2) + (B1) + (B2) + (C19) + (S2-1) (A1) + (A2) + (B1) + (B2) + (C20) + (S2-1), (A1) + (A2) + (B1) + (B2) + (C30) + (S2-1).

Es kann sinnvoll sein, eines oder mehrere Herbizide (A) mit einem oder mehreren Herbiziden (B) und einem oder mehreren Herbiziden (C) zu kombinieren, z.B. ein Herbizid (A) mit einem Herbizid (B) und einem oder mehreren Herbiziden (C). Weiterhin können die erfindungsgemäßen Herbizid-Kombinationen zusammen mit anderen agrochemischen Wirkstoffen beispielsweise aus der Gruppe der Safener, Fungizide, Herbizide, Insektizide und Pflanzenwachstumsregulatoren oder im Pflanzenschutz üblichen Zusatzstoffen und Formulierungshilfsmittel eingesetzt werden. Zusatzstoffe sind beispielsweise Düngemittel und Farbstoffe. Die oben genannten Aufwandmengenbereiche und Aufwandmengenverhältnisse sind dabei jeweils bevorzugt.

Die erfindungsgemäßen Kombinationen (= herbiziden Mittel) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Bevorzugt ist die Anwendung im Nachauflaufverfahren oder im frühen Nachsaat-Vorauflaufverfahren.

Im einzelnen seien beispielhaft einige Vertreter der mono- und dikotylen Unkrautflora genannt, die durch die erfindungsgemäßen Herbizid-Kombinationen kontrolliert werden können, ohne daß durch die Nennung eine Beschränkung auf bestimmte Arten erfolgen soll.

Auf der Seite der monokotylen Unkrautarten werden z.B. Avena spp., Alopecurus spp., Apera spica venti, Brachiaria spp., Digitaria spp., Lolium spp., Echinochloa spp., Panicum spp., Phalaris spp., Poa spp., Setaria spp. sowie Bromus spp. wie Bromus catharticus, Bromus secalinus, Bromus erectus, Bromus tectorum und Bromus japonicus und Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Abutilon spp., Amaranthus spp., Chenopodium spp., Chrysanthemum spp., Galium spp. wie Galium aparine, Ipomoea spp., Kochia spp., Lamium spp., Matricaria spp., Pharbitis spp., Polygonum spp.,Sida spp., Sinapis spp., Solanum spp., Stellaria spp., Veronica spp. und Viola spp., Xanthium spp., auf der annuellen Seite sowie Convolvulus, Cirsium, Rumex und Artemisia bei den perennierenden Unkräutern. Werden die erfindungsgemäßen Herbizid-Kombinationen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkorikurrenz sehr früh und nachhaltig beseitigt wird.

Die erfindungsgemäßen herbiziden Kombinationen zeichnen sich durch eine schnell einsetzende und lang andauernde herbizide Wirkung aus. Die Regenfestigkeit der Wirkstoffe in den erfindungsgemäßen Kombinationen ist in der Regel günstig. Als besonderer Vorteil fällt ins Gewicht, daß die in den Kombinationen verwendeten und wirksamen Dosierungen von Verbindungen (A), (B) und (C) so gering eingestellt werden können, daß ihre Bodenwirkung optimal niedrig ist. Somit wird deren Einsatz nicht nur in empfindlichen Kulturen erst möglich, sondern Grundwasser-Kontaminationen werden praktisch vermieden. Durch die erfindungsgemäßen Kombination von Wirkstoffen wird eine erhebliche Reduzierung der nötigen Aufwandmenge der Wirkstoffe ermöglicht.

Bei der gemeinsamen Anwendung von Herbiziden des Typs (A)+(B)+(C) treten in bevorzugter Ausführungsform überadditive (= synergistische) Effekte auf. Dabei ist die Wirkung in den Kombinationen stärker als die zu erwartende Summe der Wirkungen der eingesetzten Einzelherbizide. Die synergistischen Effekte erlauben eine Reduzierung der Aufwandmenge, die Bekämpfung eines breiteren Spektrums von Unkräutern und Ungräsem, einen schnelleren Einsatz der herbiziden Wirkung, eine längere Dauerwirkung, eine bessere Kontrolle der Schadpflanzen mit nur einer bzw. wenigen Applikationen sowie eine Ausweitung des möglichen Anwendungszeitraumes. Teilweise wird durch den Einsatz der Mittel auch die Menge an schädlichen Inhaltsstoffen, wie Stickstoff oder Ölsäure, und deren Eintrag in den Boden reduziert.

Die genannten Eigenschaften und Vorteile sind in der praktischen Unkrautbekämpfung von Nutzen, um landwirtschaftliche Kulturen von unerwünschten Konkurrenzpflanzen freizuhalten und damit die Erträge qualitativ und quantitativ zu sichern und/oder zu erhöhen. Der technische Standard wird durch diese neuen Kombinationen hinsichtlich der beschriebenen Eigenschaften deutlich übertroffen.

Obgleich die erfindungsgemäßen Kombinationen eine ausgezeichnete herbizide Aktivität gegenüber mono- und dikotylen Schadpflanzen aufweisen, werden die Kulturpflanzen nur unwesentlich oder gar nicht geschädigt.
Darüberhinaus weisen die erfindungsgemäßen Mittel teilweise hervorragende wachstumsregulatorische Eigenschaften bei den Kulturpflanzen auf. Sie greifen regulierend in den pflanzeneigenen Stoffwechsel ein und können damit zur gezielten Beeinflussung von Pflanzeninhaltsstoffen und zur Emteerieichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da Ernteverluste beim Lagern hierdurch verringert oder völlig verhindert werden können.

Aufgrund ihrer herbiziden und pflanzenwachstumsregulatorischen Eigenschaften können die erfindungsgemäßen Mittel zur Bekämpfung von Schadpflanzen in gentechnisch veränderten oder durch Mutationsselektion erhaltenen Kulturpflanzen eingesetzt werden. Diese Kulturpflanzen zeichnen sich in der Regel durch besondere vorteilhafte Eigenschaften aus, wie Resistenzen gegenüber herbiziden Mitteln oder Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind z.B. transgene Pflanzen mit erhöhtem Stärkegehalt oder veränderter Qualität der Stärke oder solche mit anderer Fettsäurezusammensetzung des Ernteguts bekannt.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten (siehe z.B. US 5,162,602; US 4,761,373; US 4,443,971). Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche Resistenzen gegen andere Herbizide aufweisen, beispielsweise gegen Sulfonylharnstoffe (EP-A-0257993, US-A-5013659),
- transgene Kulturpflanzen, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.
Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind. Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci, USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, z.B. sowohl monokotyle als auch dikotyle Pflanzen. So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Gegenstand der vorliegenden Erfindung ist weiterhin auch ein Verfahren zur Bekämpfung von unerwünschtem Pflanzenwuchs (z.B. Schadpflanzen), vorzugsweise in Pflanzenkulturen wie Getreide (z.B. Weizen, Gerste, Roggen, Hafer, Kreuzungen davon wie Triticale, Reis, Mais, Hirse), Zuckerrübe, Zuckerrohr, Raps, Baumwolle und Soja, besonders bevorzugt in monokotylen Kulturen wie Getreide, z.B. Weizen, Gerste, Roggen, Hafer, Kreuzungen davon wie Triticale, Reis, Mais und Hirse, wobei man ein oder mehrere Herbizide des Typs (A) mit einem oder mehreren Herbiziden des Typs (B) und einem oder mehreren Herbiziden des Typs (C), gemeinsam oder getrennt, z.B. im Vorauflauf, Nachauflauf oder im Vorauflauf und Nachauflauf auf die Pflanzen, z.B. Schadpflanzen, Pflanzenteile, Pflanzensamen oder die Fläche auf der die Pflanzen wachsen, z.B. die Anbaufläche appliziert.

Die Pflanzenkulturen können auch gentechnisch verändert oder durch Mutationsselektion erhalten sein und sind bevorzugt tolerant gegenüber Acetolactatsynthase (ALS)-Inhibitoren.

Gegenstand der Erfindung ist auch die Verwendung der neuen Kombinationen aus Verbindungen (A)+(B) + (C) zur Bekämpfung von Schadpflanzen, vorzugsweise in Pflanzenkulturen.

Die erfindungsgemäßen herbiziden Mittel können auch nicht-selektiv zur Bekämpfung unerwünschten Pflanzenwuchses eingesetzt werden, z.B. in Plantagenkulturen, an Wegrändern, Plätzen, Industrieanlagen oder Eisenbahnanlagen.

Die erfindungsgemäßen Wirkstoffkombinationen können sowohl als Mischformulierungen der Komponenten (A), (B) und (C) gegebenenfalls mit weiteren agrochemischen Wirkstoffen, Zusatzstoffen und/oder üblichen Formulierungshilfsmitteln vorliegen, die dann in üblicher Weise mit Wasser verdünnt zur Anwendung gebracht werden, oder als sogenannte Tankmischungen durch gemeinsame Verdünnung der getrennt formulierten oder partiell getrennt formulierten Komponenten mit Wasser hergestellt werden.

Die Verbindungen (A), (B) und (C) und deren Kombinationen können auf verschiedene Art formuliert werden, je nachdem welche biologischen und/oder chemisch-physikalischen Parameter vorgegeben sind. Als allgemeine Formulierungsmöglichkeiten kommen beispielsweise in Frage: Spritzpulver (WP), wasserlösliche Konzentrate, emulgierbare Konzentrate (EC), wäßrige Lösungen (SL), Emulsionen (EW) wie Öl-in-Wasser- und Wasser-in-Öl-Emulsionen, versprühbare Lösungen oder Emulsionen, Suspensionskonzentrate (SC), Dispersionen auf Öl- oder Wässerbasis, Suspoemulsionen, Stäubemittel (DP), Beizmittel, Granulate zur Boden- oder Streuapplikation oder wasserdispergierbare Granulate (WG), ULV-Formulierungen, Mikrokapseln oder Wachse.
Die einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Valkenburg, "Pesticide Formulations", Marcel Dekker N.Y., 1973; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v. Olphen, "Introduction to Clay Colloid Chemistry"; 2nd Ed., J. Wiley & Sons, N.Y. Marsden, "Solvents Guide", 2nd Ed., Interscience, N.Y. 1950; McCutcheon's, "Detergents and Emulsifers Annual", MC Publ. Corp., Ridegewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976, Winnacker-Küchler, "Chemische Technologie", Band 7, C. Hauser Verlag München, 4. Aufl. 1986.

Auf der Basis dieser Formulierungen lassen sich auch Kombinationen mit anderen agrochemischen Wirkstoffen, wie anderen Herbiziden, Fungiziden, Insektiziden, sowie Safenem, Düngemitteln und/oder Wachstumsregulatoren herstellen, z.B. in Form einer Fertigformulierung oder als Tankmix.

Spritzpulver (benetzbare Pulver) sind in Wasser gleichmäßig dispergierbar Präparate, die neben dem Wirkstoff außer einem Verdünnungs- oder Inertstoff noch Tenside ionischer oder nichtionischer Art (Netzmittel, Dispergiermittel), z.B. polyoxethylierte Alkylphenole, polyethoxylierte Fettalkohole oder -Fettamine, Alkansulfonate oder Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten.

Emulgierbare Konzentrate werden durch Auflösen des Wirkstoffs in einem organischen Lösungsmittel, z.B. Butanol, Cyclohexanon, Dimethylformamid, Xylol oder auch höhersiedenden Aromaten oder Kohlenwasserstoffe unter Zusatz von einem oder mehreren ionischen oder nichtionischen Tensiden (Emulgatoren) hergestellt. Als Emulgatoren können beispielsweise verwendet werden: Alkylarylsulfonsaure Calcium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitanfettsäureester oder Polyoxethylensorbitester.

Stäubemittel erhält man durch Vermahlen des Wirkstoffs mit fein verteilten festen Stoffen, z.B. Talkum, natürlichen Tonen, wie Kaolin, Bentonit und Pyrophyllit, oder Diatomeenerde.

Suspensionskonzentrate (SC) können auf Wasser- oder Ölbasis sein. Sie können beispielsweise durch Naß-Vermahlung mittels handelsüblicher Perlmühlen und gegebenenfalls Zusatz von weiteren Tensiden, wie sie z.B. oben bei den anderen Formulierungstypen bereits aufgeführt sind, hergestellt werden.

Emulsionen, z.B. ÖI-in-Wasser-Emulsionen (EW), lassen sich beispielsweise mittels Rührem, Kolloidmühlen und/oder statischen Mischern unter Verwendung von wäßrigen organischen Lösungsmitteln und gegebenenfalls von weiteren Tensiden, wie sie z.B. oben bei den anderen Formulierungstypen bereits aufgeführt sind, herstellen.

Granulate können entweder durch Verdüsen des Wirkstoffs auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden. Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischem und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Formulierungen enthalten in der Regel 0,1 bis 99 Gewichtsprozent, insbesondere 2 bis 95 Gew.-%, Wirkstoffe der Typen A und/oder B und/oder C, wobei je nach Formulierungsart folgende Konzentrationen üblich sind: In Spritzpulvern beträgt die Wirkstoffkonzentration z.B. etwa 10 bis 95 Gew.-%, der Rest zu 100 Gew.-% besteht aus üblichen Formulierungsbestandteilen. Bei emulgierbaren Konzentraten kann die Wirkstoffkonzentration z.B. 5 bis 80 Gew.-%, betragen. Staubförmige Formulierungen enthalten meistens 5 bis 20 Gew.-% an Wirkstoff, versprühbare Lösungen etwa 0,2 bis 25 Gew.-% Wirkstoff. Bei Granulaten wie dispergierbaren Granulaten hängt der Wirkstoffgehalt zum Teil davon ab, ob die wirksame Verbindung flüssig oder fest vorliegt und welche Granulierhilsmittel und Füllstoffe verwendet werden. In der Regel liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Konservierungs-, Frostschutz- und Lösungsmittel, Füll-, Farb- und Trägerstoffe, Entschäumer, Verdunstungshemmer und Mittel, die den pH-Wert oder die Viskosität beeinflussen.

Die herbizide Wirkung der erfindungsgemäßen Herbizid-Kombinationen kann z.B. durch oberflächenaktive Substanzen verbessert werden, vorzugsweise durch Netzmittel aus der Reihe der Fettalkohol-Polyglykolether. Die Fettalkohol-Polyglykolether enthalten vorzugsweise 10 -18 C-Atome im Fettalkoholrest und 2 - 20 Ethylenoxideinheiten im Polyglykoletherteil. Die Fettalkohol-polyglykolether können nichtionisch vorliegen, oder ionisch, z.B. in Form von Fettalkoholpolyglykolethersulfaten vorliegen, die z.B als Alkalisalze (z.B. Natrium- und Kaliumsalze) oder Ammoniumsalze, oder auch als Erdalkalisalze wie Magnesiumsalze verwendet werden, wie C₁₂/C₁₄-Fettalkohol-diglykolethersulfat-Natrium (Genapol^{®} LRO, Clariant GmbH); siehe z.B. EP-A-0476555, EP-A-0048436, EP-A-0336151 oder US-A-4,400,196 sowie Proc. EWRS Symp. "Factors Affecting Herbicidal Activity and Selectivity", 227 - 232 (1988). Nichtionische Fettalkoholpolyglykolether sind beispielsweise 2 - 20, vorzugsweise 3 - 15, Ethylenoxideinheiten enthaltende (C₁₀-C₁₈)-, vorzugsweise (C₁₀-C₁₄)-Fettalkohol-polyglykolether (z.B. Isotridecylalkohol-polyglykolether) wie Genapol^{®} X-030, Genapol^{®} X-060, Genapol^{®} X-080 oder Genapol^{®} X-150 (alle von Clariant GmbH).

Die vorliegende Erfindung umfaßt ferner die Kombination von Herbiziden (A), (B) und (C) mit den vorgängig genannten Netzmitteln aus der Reihe der Fettalkohol-Polyglykolether, die vorzugsweise 10 - 18 C-Atome im Fettalkoholrest und 2 - 20 Ethylenoxideinheiten im Polyglykoletherteil enthalten und nichtionisch oder ionisch (z.B. als Fettalkohol-polyglykolethersulfate) vorliegen können. Bevorzugt sind C₁₂/C₁₄-Fettalkohol-diglykolethersulfat-Natrium (Genapol^{®} LRO, Clariant GmbH) und Isotridecylalkohol-Polyglykolether, mit 3 - 15 Ethylenoxideneinheiten, z.B. aus der Genapol^{®} X-Reihe wie Genapol^{®} X-030, Genapol^{®} X-060, Genapol^{®} X-080 und Genapol^{®} X-150 (alle von Clariant GmbH). Weiterhin ist bekannt, daß Fettalkohol-Polyglykolether wie nichtionische oder ionische Fettalkohol-polyglykolether (z.B. Fettalkohol-Polyglykolethersulfate) auch als Penetrationshilfsmittel und Wirkungsverstärker für eine Reihe anderer Herbizide, unter anderem auch für Herbizide aus der Reihe der Imidazolinone geeignet sind (siehe z.B. EP-A-0502014).

Die herbizide Wirkung der erfindungsgemäßen Herbizid-Kombinationen kann auch durch die Verwendung von Pflanzenölen verstärkt werden. Unter dem Begriff Pflanzenöle werden Öle aus ölliefernden Pflanzenarten wie Sojaöl, Rapsöl, Maiskeimöl, Sonnenblumenöl, Baumwollsaatöl, Leinöl, Kokosöl, Palmöl, Distelöl oder Rhizinusöl, insbesondere Rapsöl verstanden, sowie deren Umesterungsprodukte, z.B. Alkylester wie Rapsölmethylester oder Rapsölethylester. Die Pflanzenöle sind bevorzugt Ester von C₁₀-C₂₂-, vorzugsweise C₁₂-C₂₀-Fettsäuren. Die C₁₀-C₂₂-Fettsäureester sind beispielsweise Ester ungesättigter oder gesättigter C₁₀-C₂₂-Fettsäuren, insbesondere mit gerader Kohlenstoffatomzahl, z.B. Erucasäure, Laurinsäure, Palmitinsäure und insbesondere C₁₈-Fettsäuren wie Stearinsäure, Ölsäure, Linolsäure oder Linolensäure.

Beispiele für C₁₀-C₂₂-Fettsäure-Ester sind Ester, die durch Umsetzung von Glycerin oder Glykol mit den C₁₀-C₂₂-Fettsäuren erhalten werden, wie sie z.B. in Ölen aus ölliefernden Pflanzenarten enthalten sind, oder C₁-C₂₀-Alkyl-C₁₀-C₂₂-Feftsäure-Ester, wie sie z.B. durch Umesterung der vorgenannten Glycerin- oder Glykol-C₁₀-C₂₂-Fettsäure-Ester mit C₁-C₂₀-Alkoholen (z.B. Methanol, Ethanol, Propanol oder Butanol) erhalten werden können. Die Umesterung kann nach bekannten Methoden erfolgen, wie sie z.B. beschrieben sind im Römpp Chemie Lexikon, 9. Auflage, Band 2, Seite 1343, Thieme Verlag Stuttgart.

Als C₁-C₂₀-Alkyl-C₁₀-C₂₂-Fettsäure-Ester bevorzugt sind Methylester, Ethylester, Propylester, Butylester, 2-ethyl-hexylester und Dodecylester. Als Glykol- und Glycerin-C₁₀-C₂₂-Fettsäure-Ester bevorzugt sind die einheitlichen oder gemischten Glykolester und Glycerinester von C₁₀-C₂₂-Fettsäuren, insbesondere solcher Fettsäuren mit gerader Anzahl an Kohlenstoffatomen, z.B. Erucasäure, Laurinsäure, Palmitinsäure und insbesondere C₁₈-Fettsäuren wie Stearinsäure, Ölsäure, Linolsäure oder Linolensäure.

Die Pflanzenöle können in den erfindungsgemäßen herbiziden Mitteln z.B. in Form kommerziell erhältlicher ölhaltiger Formulierungszusatzstoffe, insbesondere solcher auf Basis von Rapsöl wie Hasten^{®} (Victorian Chemical Company, Australien, nachfolgend Hasten genannt, Hauptbestandteil: Rapsölethylester), Actirob^{®}B (Novance, Frankreich, nachfolgend ActirobB genannt, Hauptbestandteil: Rapsölmethylester), Rako-Binol^{®} (Bayer AG, Deutschland, nachfolgend Rako-Binol genannt, Hauptbestandteil: Rapsöl), Renol^{®} (Stefes, Deutschland, nachfolgend Renol genannt, Pflanzenölbestandteil: Rapsölmethylester) oder Stefes Mero^{®} (Stefes, Deutschland, nachfolgend Mero genannt, Hauptbestandteil: Rapsölmethylester) enthalten sein.

Die vorliegende Erfindung umfasst in einer weiteren Ausführungsform Kombinationen von Herbiziden (A), (B) und (C) mit den vorgängig genannten Pflanzenölen wie Rapsöl, bevorzugt in Form kommerziell erhältlicher ölhaltiger Formulierungszusatzstoffe, insbesondere solcher auf Basis von Rapsöl wie Hasten^{®} (Victorian Chemical Company, Australien, nachfolgend Hasten genannt, Hauptbestandteil: Rapsölethylester), Actirob^{®}B (Novance, Frankreich, nachfolgend ActirobB genannt, Hauptbestandteil: Rapsölmethylester), Rako-Binol^{®} (Bayer AG, Deutschland, nachfolgend Rako-Binol genannt, Hauptbestandteil: Rapsöl), Renol^{®} (Stefes, Deutschland, nachfolgend Renol genannt, Pflanzenölbestandteil: Rapsölmethylester) oder Stefes Mero^{®} (Stefes, Deutschland, nachfolgend Mero genannt, Hauptbestandteil: Rapsölmethylester).

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Boden- bzw. Streugranulate, sowie versprühbare Lösungen werden vor der Anwendung üblicherweise nicht mehr mit weiteren inerten Stoffen verdünnt.

Die Wirkstoffe können auf die Pflanzen, Pflanzenteile, Pflanzensamen oder die Anbaufläche (Ackerboden) ausgebracht werden, vorzugsweise auf die grünen Pflanzen und Pflanzenteile und gegebenenfalls zusätzlich auf den Ackerboden. Eine Möglichkeit der Anwendung ist die gemeinsame Ausbringung der Wirkstoffe in Form von Tankmischungen, wobei die optimal formulierten konzentrierten Formulierungen der Einzelwirkstoffe gemeinsam im Tank mit Wasser gemischt und die erhaltene Spritzbrühe ausgebracht wird.

Eine gemeinsame herbizide Formulierung der erfindungsgemäßen Kombination an Wirkstoffen (A), (B) und (C) hat den Vorteil der leichteren Anwendbarkeit, weil die Mengen der Komponenten bereits im richtigen Verhältnis zueinander eingestellt sind. Außerdem können die Hilfsmittel in der Formulierung aufeinander optimal abgestimmt werden.

### A. Formulierungsbeispiele allgemeiner Art

a) Ein Stäubemittel wird erhalten, indem man 10 Gew.-Teile eines Wirkstoffs/Wirksstoffgemischs und 90 Gew.-Teile Talkum als Inertstoff mischt und in einer Schlagmühle zerkleinert.
b) Ein in Wasser leicht dispergierbares, benetzbares Pulver wird erhalten, indem man 25 Gew.-Teile eines Wirkstoffs/Wirksstoffgemischs, 64 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile eines Wirkstoffs/Wirksstoffgemischs mit 6 Gew.-Teilen Alkylphenolpolyglykolether (7Triton^{®} X 207), 3 Gew.-Teilen Isotridecanolpolyglykolether (8 EO) und 71 Gew.-Teilen paraffinischem Mineralöl (Siedebereich z.B. ca. 255 bis 277EC) mischt und in einer Reibkugelmühle auf eine Feinheit von unter 5 Mikron vermahlt.
d) Ein emulgierbares Konzentrat wird erhalten aus 15 Gew.-Teilen eines Wirkstoffs/Wirksstoffgemischs, 75 Gew.-Teilen Cyclohexanon als Lösemittel und 10 Gew.-Teilen oxethyliertem Nonylphenol als Emulgator.
e) Ein in Wasser dispergierbares Granulat wird erhalten indem man
   75 Gew.-Teile eines Wirkstoffs/Wirksstoffgemischs,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Gränuliefflüssigkeit granuliert.
f) Ein in Wasser dispergierbares Granulat wird auch erhalten, indem man
   25 Gew.-Teile eines Wirkstoffs/Wirksstoffgemischs,
   5 Gew.-Teile 2,2'-dinaphthylmethan-6,6'-disulfonsaures Natrium,
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstöffdüse zerstäubt und trocknet.

### B. Biologische Beispiele

### Herbizide Wirkung und Verträglichkeit in Feldversuchen

Auf Versuchsfeldern mit natürlicher Verunkraütung wurden die Pflanzen praxisüblich ausgesät und gepflegt (Düngung, Beregnung etc.).

Die Versuche wurden in randomisierten Versuchsanlagen aus 2 Parzellen von 5-10 m² bei 2-3 facher Wiederholung angelegt. Die Applikation erfolgte mit Parzellenspritzgeräten im 3-5 Blattstadium. Dabei wurden die Prüfsubstanzen in 100 - 600 Liter Wasser / ha angesetzt und mit 3 bar Druck ausgebracht.

Nach der Anwendung (ca. 4 Wochen nach Applikation) wurde die herbizide Wirksamkeit der Wirkstoffe bzw. Wirkstoffmischungen anhand der behandelten Pflanzen im Vergleich zu unbehandelten Kontrollen visuell bonitiert. Dabei wurde die Schädigung und Entwicklung aller oberirdischen Pflanzenteile erfaßt. Die Bonitierung erfolgte nach einer Prozentskala (100% Wirkung = alle Pflanzen abgestorben; 50 % Wirkung = 50% der Pflanzen und grünen Pflanzenteile abgestorben; 0 % Wirkung = keine erkennbare Wirkung bzw. Schädigung an Kulturarten).

Die Ergebnisse sind in den nachfolgenden Tabellen als Mittelwerte von 2-3 Wiederholungen angegeben, wobei in Klammern die Wirkung bei unabhängiger Anwendung der Wirkstoffe (A + B) und C) angegeben ist.

### Beispiele (nicht erfindungsgemäß)

| | | gAS/ ha | Winter-Weizen | Galium aparine | Avena fatua |
|---|---|---|---|---|---|
| | | | % Schädigung | % Wirkung | |
| (A+B) | (A1 + B2)^{s} | 12,5 + 6,25 | 0 | 55 | 25 |
| C) | Azur®: C22) + C23) + C37) | 800 + 40 + 200 | 0 | 35 | 65 |
| (A + B) + C) | | (12,5 + 6,25) + (800 + 40 + 200) | 0 | 96 (55 + 35) | 93 (25 + 65) |
| D) | B2) | 5 | 0 | 25 | 68 |
| (A + B) + D) | | (12,5 + 6,25) + 5 | 0 | 88 (55 + 25) | 96 (25 + 68) |
| E) | C58) | 60 | 0 | 0 | 70 |
| (A + B) + E) | | (12,5 + 6,25) + 60 | 0 | 65 (55 + 0) | 97 (25 + 70) |

| | | | | | |
|---|---|---|---|---|---|
| Abkürzungen: gAS/ha = Aufwandmenge in Gramm Aktivsubstanz pro Hektar (A1+B2)^{s} =Amidosulfuron + Iodosulfuron-methyl-Natrium + Mefenpyr-diethyl (S1-1) C22 = Bromoxynil C23 = Isoproturon C 37 = Diflufenican C 58 = Fenoxaprop-(P)-ethyl | | | | | |

## Patentansprüche

1. Herbizid-Kombination mit einem synergistisch wirksamen Gehalt an Komponenten (A), (B) und (C), wobei
(A) ein oder mehrere Herbizide aus der Gruppe der Verbindungen der Formel (I) und deren Salzen bedeutet, 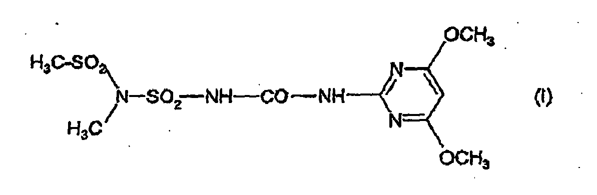
(B) ein oder mehrere Herbizide aus der Gruppe der Verblhdungen der Formel (II) und deren Salzen bedeutet, 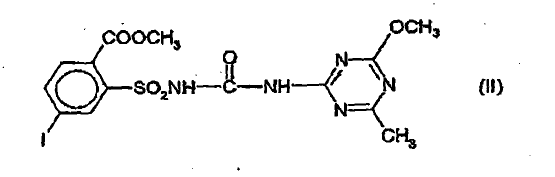
(C) bedeutet ein oder mehrere selektiv in einigen monokotylen Kulturen gegen monokotyle und/oder dikotyle Schadpflanzen wirksamen Herbizide aus der Gruppe der Herbizide, bestehend aus
(C1) Flucarbazone
(C2) Procarbazone
C5 Tritosulfuron
(C13) Flufenacet
(C19) Sulfosulfuron
(C20) Flupyrsulfuron-methyl
(C30) MCPB

2. Herbizid-Kombination nach Anspruch 1, worin als Komponenten (A) Amidosulfuron und/oder Amidosulfuron-Natrium und als Komponente (B) Iodosulfuron-methyl und/oder Iodosulfuron-methyl-Natrium enthalten ist.

3. Herbizid-Kombination nach Anspruch 1 oder 2, zusätzlich enthaltend eine oder mehrere weitere Komponenten aus der Gruppe enthaltend agrochemische Wirkstoffe anderer Art, im Pflanzenschutz übliche Zusatzstoffe und Formulierungshilsmlttel.

4. Herbizid-Kombination nach einem oder mehreren der Ansprüche 1 - 3, zusätzlich enthaltend einen oder mehrere Safener.

5. Herbizid-Kombination nach einem oder mehreren der Ansprüche 1 - 4, zusätzlich enthaltend einen oder mehrere Fettalkohol-Polyglykolether und/oder eines oder mehrere Pflanzenöle.

6. Herbizid-Kombination gemäß Anspruch 5, worin das Pflanzenöl ein Rapsöl ist.

7. Herbizid-Kombination gemäß Anspruch 5 oder 6, worin der Fettalkohol-Polyglykolether ein nichtionischer oder Ionischer Fettalkohol-Polyglykolether ist.

8. Verfahren zur Bekämpfung von unerwünschtern Pflanzenwuchs, worin die Herbizide (A), (B) und (C), definiert gemäß Anspruch 1 oder 2, gemeinsam oder getrennt auf die Pflanzen, Pflanzenteile, Pflanzensamen oder die Fläche, auf der die Pflanzen wachsen, appliziert werden.

9. Verfahren nach Anspruch 8 zur selektiven Bekämpfung von Schadpflazen in Pflanzenkultufien..

10. Verfahren nach Anspruch 9 zur Bekämpfung von Schadpflanzen in monokotylen Pflanzenkulturen.

11. Verfahren nach Anspruch 9 oder 10, worin die Pflanzenkulturen gentechnisch verändert oder durch Mutationsselektion erhalfen sind.

12. Verwendung der nach einem der Ansprüche 1 bis 7 definierten Herbizid-Kombination zur Bekämpfung von Schadpflanzen.

## Claims

1. A herbicide combination comprising a synergistically effective amount of components (A), (B) and (C), where
(A) denotes one or more herbicides selected from the group of the compounds of the formula (I) and their salts 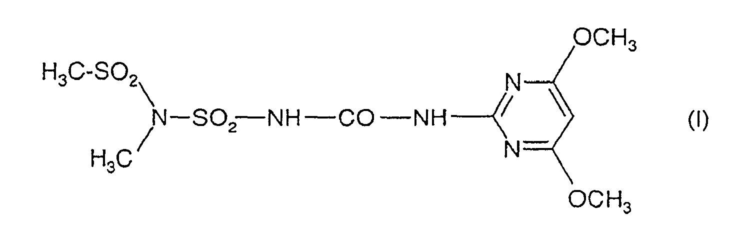
(B) denotes one or more herbicides selected from the group of the compounds of the formula (II) and their salts 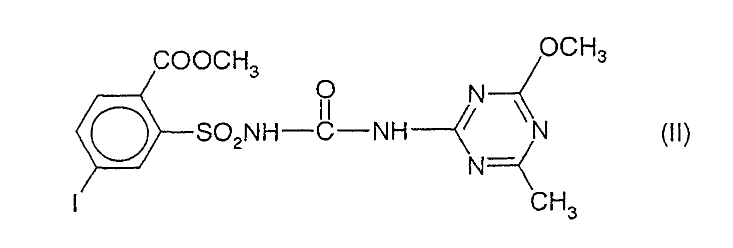
(C) denotes one or more herbicides which act selectively in some monocotyledonous crops against monocotyledonous and/or dicotyledonous harmful plants, which herbicides are selected from the group of herbicides consisting of
(C1) flucarbazone
(C2) procarbazone
(C5) tritosulfuron
(C13) flufenacet
(C19) sulfosulfuron
(C20) flupyrsulfuron - methyl
(C30) MCPB

2. The herbicide combination as claimed in claim 1 which comprises, as component (A), amidosulfuron and/or amidosulfuron-sodium and, as component (B), iodosulfuron-methyl and/or iodosulfuron-methyl sodium.

3. The herbicide combination as claimed in claim 1 or 2 which additionally comprises one or more further components selected from the group consisting of agrochemically active compounds of a different type, formulation auxiliaries and additives customary in crop protection.

4. The herbicide combination as claimed in one or more of claims 1 - 3 which additionally comprises one or more safeners.

5. The herbicide combination as claimed in one or more of claims 1-4, which additionally comprises one or more fatty alcohol polyglycol ethers and/or one or more vegetable oils.

6. The herbicide combination as claimed in claim 5, where the vegetable oil is a rapeseed oil.

7. The herbicide combination as claimed in claim 5 or 6, where the fatty alcohol polyglycol ether is a nonionic or ionic fatty alcohol polyglycol ether.

8. A method for controlling unwanted vegetation, which comprises applying the hebicides (A), (B) and (C), defined according to claim 1 or 2, jointly or separately onto the plants, parts of plants, plant seeds or the area where the plants grow.

9. The method as claimed in claim 8 for the selective control of harmful plants in plants crop.

10. The method as claimed in claim 9 for the control of harmful plants in monocotyledonous crop plants.

11. The method as claimed in claim 9 or 10 in which the crop plants are genetically modified or have been obtained by mutation selection.

12. The use of the herbicide combination defined in any of claims 1 to 7 for controlling harmful plants.

## Revendications

1. Combinaison d'herbicides ayant une teneur à effet synergique de composants (A), (B) et (C), où
(A) désigne un ou plusieurs herbicides du groupe des composés de formule (I) et de leurs sels 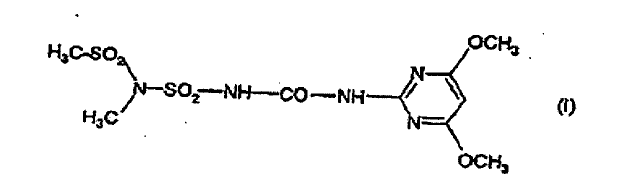
(B) désigne un ou plusieurs herbicides du groupe des composés de formule (II) et de leurs sels 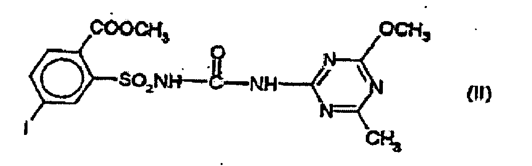
(C) représente un ou plusieurs herbicides agissant sélectivement dans certaines cultures monocotylédones contre des plantes parasites monocotylédones et/ou dicotylédones, du groupe des herbicides constitué de
(C1) flucarbazone
(C2) procarbazone
(C5) tritosulfuron
(C13) flufénacet
(C19) sulfosulfuron
(C20) flupyrsulfuron-méthyle
(C30) MCPB.

2. Combinaison d'herbicides suivant la revendication 1, dans laquelle sont contenus de l'amidosulfuron et/ou de l'amidosulfuron-sodium comme composant (A) et de l'iodosulfuron-méthyle et/ou de l'iodosulfuron-méthyl-sodium comme composant (B).

3. Combinaison d'herbicides suivant la revendication 1 ou 2, contenant en outre un ou plusieurs autres composants choisis dans le groupe comprenant des substances actives agrochimiques d'une autre catégorie, des additifs et des auxiliaires de formulation d'emploi courant dans la protection des plantes.

4. Combinaison d'herbicides suivant une ou plusieurs des revendications 1 - 3, contenant en outre un ou plusieurs agents phytoprotecteurs.

5. Combinaison d'herbicides suivant une ou plusieurs des revendications 1 - 4, contenant en outre un ou plusieurs éthers de polyglycols d'alcools gras et/ou une ou plusieurs huiles végétales.

6. Combinaison d'herbicides suivant la revendication 5, dans laquelle l'huile végétale est une huile de colza.

7. Combinaison d'herbicides suivant la revendication 5 ou 6, dans laquelle l'éther de polyglycol d'alcool gras est un éther de polyglycol d'alcool gras non ionique ou ionique.

8. Procédé de lutte contre une végétation indésirable, dans lequel les herbicides (A), (B) et (C) définis suivant la revendication 1 ou 2 sont appliqués conjointement ou séparément sur les plantes, des parties de plantes, des semences de plantes ou l'aire sur laquelle les plantes croissent.

9. Procédé suivant la revendication 8, destiné à la lutte sélective contre des plantes parasites dans des cultures de végétaux.

10. Procédé suivant la revendication 8, destiné à la lutte contre des plantes parasites dans des cultures de plantes monocotylédones.

11. Procédé suivant la revendication 9 ou 10, dans lequel les plantes cultivées sont modifiées par une technique génétique ou obtenues par une sélection par mutation.

12. Utilisation de la combinaison d'herbicides définie suivant l'une des revendications 1 à 7 pour la lutte contre des plantes parasites.
